Europäisches Patentamt

European Patent Office

Office européen des brevets

(19)

(11) **EP 1 510 171 A1**

(12) **EUROPEAN PATENT APPLICATION**

(43) Date of publication:
**02.03.2005 Bulletin 2005/09**

(51) Int Cl.⁷: **A61B 5/00**, A61B 5/15

(21) Application number: **04255207.5**

(22) Date of filing: **27.08.2004**

(84) Designated Contracting States:
**AT BE BG CH CY CZ DE DK EE ES FI FR GB GR
HU IE IT LI LU MC NL PL PT RO SE SI SK TR**
Designated Extension States:
**AL HR LT LV MK**

(30) Priority: **28.08.2003 US 652464
28.08.2003 US 653023**

(71) Applicant: **LIFESCAN, INC.
Milpitas, California 95035 (US)**

(72) Inventors:
• **Stout, Phil
Roseville, MN 55113 (US)**
• **Melander, Todd
Minneapolis, MN 55408 (US)**

(74) Representative: **Mercer, Christopher Paul et al
Carpmaels & Ransford,
43-45 Bloomsbury Square
London WC1A 2RA (GB)**

(54) **Analytical device with prediction module and related methods**

(57)     An analytical device for predicting a subject's whole blood analyte concentration based on the subject's interstitial fluid (ISF) analyte concentration includes an ISF sampling module, an analysis module and a prediction module. The ISF sampling module is configured to sequentially extract a plurality of ISF samples from a subject. The analysis module is configured to sequentially determining an ISF analyte concentration (e. g., ISF glucose concentration) in each of the ISF samples, resulting in a series of ISF analyte concentrations. The prediction module is configured for storing the series of ISF analyte concentrations and predicting the subject's whole blood analyte concentration based on the series by performing at least one algorithm. A method for predicting a subject's whole blood analyte concentration based on the subject's interstitial fluid analyte concentration includes extracting a plurality of interstitial fluid (ISF) samples from a subject in a sequential manner and sequentially determining an ISF analyte concentration in each of the plurality of ISF samples to create a series of ISF analyte concentrations. The subject's blood analyte concentration is then predicted based on the series of ISF analyte concentrations by performing at least one algorithm.

EP 1 510 171 A1

**Description**

**BACKGROUND OF INVENTION**

This application is a continuation-in-part of U.S. Application No. 10/653,023, filed August 28, 2003, which claims the benefit of U.S. Provisional Application No. 60/476,733, filed June 6, 2003.

1. Field of the Invention

The present invention relates, in general, to analytical devices and, in particular, to analytical devices and associated methods for predicting a subject's blood analyte concentration from a subject's interstitial fluid (ISF) analyte concentration.

Description of the Related Art

In the field of analyte (e.g., glucose) monitoring, continuous or semi-continuous analytical devices and methods are advantageous in that they provide enhanced insight into analyte concentration trends, a subject's overall analyte control and the effect of food, exercise and/or medication on an analyte's concentration. In practice, however, such analytical devices can have drawbacks. For example, interstitial fluid (ISF) analytical devices can suffer inaccuracies due to, for instance, physiological lag (i.e., the time-dependent difference between a subject's ISF analyte concentration and a subject's blood analyte concentration) and/or bias effects (i.e., the fluid characteristic-dependent difference between a subject's ISF analyte concentration and a subject's blood analyte concentration).
Conventional ISF analytical devices can employ ISF samples obtained from various sites on a subject's body and from various penetration depths in a subject's skin. The use of various sites and penetration depths for obtaining an ISF sample can be a contributing factor in an ISF analytical devices' inaccuracy. In addition, other analytically relevant properties of an ISF sample can be influenced by the site and/or penetration depth at which the ISF sample is collected. For example, ISF collected from the subcutaneous region of a subject's skin can be more prone to containing contaminating substances such as triglycerides, which can affect analyte analysis in terms of volume error and sensor fouling.
Furthermore, conventional ISF analytical devices can require inconvenient and cumbersome calibration procedures involving samples of capillary blood.
Still needed in the field, therefore, is an analytical device and associated method with reduced inaccuracy due to physiological lag and bias effects. In addition, the analytical device and associated methods should not require samples of capillary blood for calibration.

**SUMMARY OF INVENTION**

Embodiments of the present invention include analytical devices and methods that accurately account for physiological lag and bias effects. In addition, the analytical device and associated methods do not require samples of capillary blood for calibration.
An analytical device for predicting a subject's whole blood analyte concentration based on the subject's interstitial fluid (ISF) analyte concentration according to an exemplary embodiment of the present invention includes an ISF sampling module, an analysis module and a prediction module.
The ISF sampling module is configured to extract a plurality of ISF samples from a subject in a sequential manner. The analysis module is configured to sequentially determining an ISF analyte concentration (e.g., ISF glucose concentration) in each of the plurality of ISF samples. The result of this sequential determination is a series of ISF analyte concentrations. The prediction module is configured for storing the series of ISF analyte concentrations and predicting the subject's whole blood analyte concentration based on the series of ISF analyte concentrations by performing at least one algorithm.
An exemplary embodiment of a method for predicting a subject's whole blood analyte concentration based on the subject's interstitial analyte concentration according to the present invention includes extracting a plurality of interstitial fluid (ISF) samples from a subject in a sequential manner and determining an ISF analyte concentration in each of the plurality of ISF samples in a sequential manner to create a series of ISF analyte concentrations. The subject's blood analyte concentration is then predicted based on the series of ISF analyte concentrations by performing at least one algorithm.
Embodiments of analytical devices and methods according to the present invention predict a subject's blood analyte concentration based solely on a series of ISF analyte concentrations derived from ISF samples extracted in a continuous or semi-continuous manner. The analytical devices and methods do so using an algorithm that predicts the subject's blood analyte concentration based on the series of ISF analyte concentrations. The algorithm accounts for physiological lag and bias effects. In addition, the analytical device does not require calibration using capillary blood.

**BRIEF DESCRIPTION OF DRAWINGS**

A better understanding of the features and advantages of the present invention will be obtained by reference to the following detailed description that sets forth illustrative embodiments, in which the principles of the invention are utilized, and the accompanying drawings of which:

FIG. 1 is a block diagram of an analytical device for predicting a subject's whole blood analyte concentration based on the subject's interstitial fluid (ISF) analyte concentration according to an exemplary embodiment of the present invention;
FIG. 2 is a Clarke Error Grid Plot for interpolated finger blood glucose (reference) versus ISF glucose concentration $(ISF_0)$;
FIG. 3 is a Clarke Error Grid Plot for interpolated finger blood glucose versus predicted finger blood glucose for an algorithm (i.e., Eqn 1) that can be employed in analytical devices and methods according to the present invention;
FIG. 4 is a Clarke Error Grid Plot for interpolated finger blood glucose versus predicted finger blood glucose for another algorithm (i.e., Eqn 2) that can be employed in analytical devices and methods according to the present invention; and
FIG. 5 is a flow chart illustrating a sequence of steps in a process according to an exemplary embodiment of the present invention.

**DETAILED DESCRIPTION OF THE INVENTION**

FIG. 1 is a block diagram of an analytical device 100 (within the dashed lines) for predicting a subject's whole blood analyte concentration based on the subject's interstitial fluid (ISF) analyte concentration according to an exemplary embodiment of the present invention. Analytical device 100 includes an interstitial fluid (ISF) sampling module 110, an analysis module 120 and a prediction module 130. The arrows of FIG. 1 indicate operative communication between the ISF sampling, analysis and prediction modules.
ISF sampling module 110 is configured to extract a plurality of ISF samples from the subject in a sequential manner and to deliver the ISF samples to analysis module 120. ISF sampling module 110 can, for example, extract ISF samples in a sequential manner with a time interval between samples in the range of 5 minutes to 15 minutes.
Analysis module 120 is adapted for sequentially determining an ISF analyte concentration in each of the plurality of ISF samples extracted by ISF sampling module 110. The result of such a sequential determination is a series of ISF analyte concentrations. Such a series of ISF analyte concentrations will typically include a plurality of ISF analyte concentrations (also referred to as "values"), each associated with a time that corresponds to the time at which the ISF sample was extracted by ISF sampling module 110. Analysis module 120 can also be configured to transfer the series of ISF analyte concentrations to prediction module 130, either individually or as a group.
Interstitial sampling module 110 can take any suitable form known to one skilled in the art including, but not limited to, sampling modules and ISF extraction devices described in co-pending U.S. Provisional Patent Application No.60/476,733 (filed June 6, 2003), and sampling modules described in International Application PCT/GB01/05634 (as published as International Publication No. WO 02/49507 A1 on June 27, 2002), both of which are hereby fully incorporated herein by reference.
Furthermore, analysis module 120 can also take any suitable form known to one skilled in the art including those described in co-pending U.S. Provisional Patent Application No. 60/476,733 (filed June 6, 2003) and in International Application PCT/GBO1/05634 (published as International Publication No. WO 02/49507 A1 on June 27, 2002). In addition, the analysis module can include any suitable analyte sensor known to those of skill in the art including, but not limited to, glucose analyte sensors based on photometric or electrochemical analytical techniques.
Prediction module 130 is configured for storing the series of ISF analyte concentrations created by the analysis module and predicting the subject's whole blood analyte concentration by performing at least one algorithm of the following general form (referred to as **Eqn 1**):

$$PC = f\,(ISF_i^{\,k},\ rate_j,\ \text{significant interaction terms}) \qquad \text{(Eqn 1)}$$

where:

PC is a predicted subject's whole blood analyte concentration;

i is an integer with predetermined values selected from the values of, for example, 0, 1, 2, 3, 4 and 5;

j is an integer with predetermined values selected from the values of, for example, 1, 2, 3, 4 and 5;

k is an integer(s) with predetermined values selected from the values of, for example, 1 and 2;

$ISF_i$ is a measured ISF analyte concentration in the series of ISF analyte concentrations, with the subscript (i) indicating which ISF value is being referred to, i.e., i = 0 indicates the most recently measured ISF analyte, i = 1 indicates one value back in the series of ISF analyte concentrations, 2 = two values back in the series of ISF analyte concentrations, etc.;

$rate_j$ is the rate of change between immediately adjacent ISF analyte concentrations in the series of ISF analyte concentrations (calculated as the difference between the immediately adjacent ISF concentrations divided by the time difference between when the immediately adjacent ISF concentrations were measured by the analysis module) with the subscript (j) referring to which immediately adjacent ISF values are used to calculate the rate, i.e., when j = 1 indicates the rate between current ISF value and the previous ISF value, j = 2 is indicative of the rate between the ISF values one previous and two previous relative to the current ISF value, etc.; and

significant interaction terms = interaction terms involving at least two of $ISF_i^k$, $rate_j$.

The mathematical form of the function ($f$) employed in Eqn 1 can be any suitable mathematical form that accounts for physiological lag between ISF analyte concentration and blood analyte concentration as well as any bias effect between ISF and blood analyte concentrations. However, it has been determined that such a relationship is suitably accurate when measured ISF analyte concentrations (i.e., $ISF_i^k$), rates ($rate_j$) and interaction terms are included. The use of rates is particularly beneficial in providing an accurate algorithm, and hence an accurate analytical device, since the time interval between the ISF analyte concentrations can be non-uniform (e.g., the time interval could vary between 5 minutes and 15 minutes).

The form of the function ($f$) can determined by, for example, a least squares regression analysis of a statistically relevant number of ISF analyte concentrations and associated blood analyte concentrations. Those skilled in the art will appreciate that any number of mathematical methods (e.g., mathematical modeling methods) can be used to analyze such data and arrive at a suitable function ($f$). For example, linear and polynomial regression analysis, time series analysis, or neural networks can be used. In the circumstance that the analyte is glucose, ISF glucose concentrations and blood glucose concentrations can be determined from ISF and blood samples extracted from diabetic subjects that have ingested glucose.

If desired, a suitable algorithm can be obtained using a mathematical modeling method that includes weighting factors to provide for greater accuracy at lower analyte concentrations values, to account for curvature in the response, and/or to account for noise in the modeled data. Weighting of input observations can also be similarly beneficial in such mathematical modeling methods.

The determination of suitable weighting factors can be, for example, an iterative process in which a weighting factor(s) is applied in a model, the weighting factor's effect on model results observed, and the weighting factor(s) adjusted based on model error reduction. The choice of weighting factors in the mathematical modeling method can also be determined, for example, by the relative importance of data ranges and/or trending direction. For example, when glucose is the analyte of interest, greater accuracy for the low end of the physiological glucose concentration range may be deemed important, and thus a weighting factor that enhances the importance of lower glucose concentrations can be employed. Such an enhancement can be accomplished, for example, by multiplying observed glucose concentrations by the inverse of the observed value raised to a predetermined power. Similarly, weighting factors can be determined which will enhance the importance of certain events or trends in observed values, such as a magnitude of the gradient of an observed rate and/or a change in direction. Furthermore, prospective weighting factors can also be arbitrarily chosen with suitable weighting factors chosen from the prospective weighting factors based on their effect on model error reduction.

Prediction module 130 can take any suitable form known to one skilled in the art including, but not limited to, the remote control modules described in co-pending U.S. Provisional Patent Application No. 60/476,733 (filed June 6, 2003), which is hereby fully incorporated herein by reference.

As an alternative to the use of **Eqn 1** above, prediction module 130 can be configured for storing the series of ISF analyte concentrations created by the analysis module and predicting the subject's whole blood analyte concentration by performing at least one algorithm of the following general form (referred to as **Eqn 2**):

$$PC = f\,(ISF_i^k,\ rate_j,\ ma_n rate_m^p,\ \text{significant interaction terms}) \qquad \text{(Eqn 2)}$$

where:

n and m are integers with predetermined values selected from the values of, for example, 1, 2 and 3;

p is an integer(s) with predetermined values selected from the values of, for example, 1 and 2; and

$ma_nrate_m$ is the moving average rate between immediately adjacent averages of groupings of ISF values, with the subscript (n) referring to the number of ISF values included in the moving average and the subscript (m) referring to the time position of the adjacent average values relative to the current values as follows (with a further explanation in the next paragraph):

n+1 = the number of points used in the moving average rate;

m-1 = first point included in the moving average. If m-1 = 0, then the current ISF value is used as the first point in the moving average calculation.

n + m always adds up to the number of points back (or removed from the current ISF value) that will be needed for calculating the moving average calculation.

n and m are integers with predetermined values selected from the values of, for example, 1, 2 and 3; and

significant interaction terms = interaction terms involving at least two of $ISF_i^k$, $rate_j$, and $ma_nrate_m^p$.

The following example illustrates the concept of the moving average rate ($ma_nrate_m$) employed in **Eqn 2** above. For the exemplary moving average rate $ma_1rate_1$, the moving average rate is a 2-point moving average rate (since m+n = 1+1 = 2) with the moving average rate calculated between the average of the grouping that includes the most recent ISF concentration and the ISF concentration one point back and the average of the grouping that includes the ISF concentrations one and two points prior to the most recent ISF concentration.

**Eqn 2** includes moving average rates (i.e., $ma_nrate_m$) to smooth the data (i.e., the series of ISF analyte concentrations and/or rates) with respect to both rate and the trending direction of an analyte concentration, thereby removing noise from the data and increasing the analytical device's accuracy. Although significant (i.e., major) changes in adjacent ISF values can be regarded as important in terms of algorithm accuracy, significant changes can also be due to noise that can adversely effect an algorithm's accuracy. The moving average rate, which is the rate of change between the means of adjacent (and overlapping) groupings of ISF values, dampens noise caused by outlier values that do not represent a true trend in the data.

Examples of suitable algorithms and the techniques used to derive the algorithms are includes in the examples below.

**Example 1: Predictive Algorithm for a Glucose Analytical Device Utilizing $ISF_i^k$, $rate_j$, and Significant Interaction Terms**

A data set (i.e., a series of ISF glucose concentrations) was generated using an experimental ISF sampling and analysis modules. The ISF sampling module and analysis module employed to generate the data set were configured to extract an ISF sample from a subject's dermal layer of skin (i.e., dermis), for example from a subject's forearm, and to measure the glucose concentration in the ISF sample. The ISF sampling module and analysis module were an integrated unit comprising a one-piece sampling module and a modified OneTouch® Ultra glucose meter with test strip. The sampling utilized a 30-gauge cannula and a penetration depth of about 1 to 2 mm. It should be noted that an ISF sample collected from the dermis is considered to have a beneficially reduced physiological lag in comparison to an ISF sample collected from a subcutaneous layer due to the dermis being closer to vascular capillary beds than the subcutaneous layer.

The ISF sampling module extracted an approximately 1 μL ISF sample from a subject's dermis via the cannula and deposited the ISF sample automatically and directly into a measurement zone of the test strip. After a brief electrochemistry development period, the meter displayed the ISF glucose concentration.

Prior to the ISF samples being extracted, 2 to 4 pounds of pressure was applied to a subject's dermis for 30 seconds, followed by a 5 minute waiting period to allow blood to perfuse (flow into) the sampling area from which the ISF would be extracted. This elevated blood-flow in the sampling area has the desirable effect of mitigating the physiological lag between blood glucose concentration and ISF glucose concentration, simply because the sampling area is better perfused with flowing blood.

Finger stick blood glucose measurements in mg/dL (i.e., blood glucose concentrations) were taken from 20 subjects, followed by measurements of glucose in forearm interstitial fluid (i.e., ISF glucose concentrations) as described above. The finger stick blood measurements were taken approximately 15 minutes apart and each was followed approximately 5 minutes later by an ISF sample extraction and ISF glucose concentration measurement.

Approximately thirty (30) pairs of observations (i.e., pairs of blood glucose concentration measurements and ISF glucose concentration measurements) were obtained for each of the 20 subjects. The observations were collected over the course of one day for each subject, in whom a change in glucose concentration was induced through the ingestion of 75g of glucose. The blood glucose concentration for each observation represents a finger stick draw occurring approximately five minutes prior to the ISF draw. Blood glucose concentration at the time of the ISF sample extraction was, therefore, linearly interpolated, with the linearly interpolated value used as a response variable in developing the algorithm below. The final ISF glucose concentration for each subject was excluded during the development of the algorithm due to the inability to accurately interpolate a blood glucose concentration.

An algorithm of the form identified above as Eqn 2 was developed from the data set using multiple linear regression. The algorithm thus developed weighted lower ISF analyte concentrations more heavily, primarily due to the relative importance of accurately predicting glucose at lower concentrations. The weight used was $ISF^{-4}$. In the absence of such weighting, higher ISF glucose concentration values produced undesirable variability in the residuals.

The parameters, estimates, errors, t-values and Pr values for the model were as follows:

| Parameter | Estimate | Error | t value | Pr > |t| |
|---|---|---|---|---|
| $ISF_0$ | 0.964114574 | 0.00900642 | 107.05 | <.0001 |
| rate2 | 3.564454310 | 0.79143125 | 4.50 | <.0001 |
| rate2*rate1 | 1.032526146 | 0.27684343 | 3.73 | 0.0002 |
| rate3 | 2.115098810 | 0.57252187 | 3.69 | 0.0002 |
| rate1*rate3 | 0.728563905 | 0.32507567 | 2.24 | 0.0255 |
| rate2*rate3 | 0.993732089 | 0.36293636 | 2.74 | 0.0064 |
| rate4 | 2.620714810 | 0.48033895 | 5.46 | <.0001 |
| rate2*rate4 | 1.149236162 | 0.38075990 | 3.02 | 0.0027 |
| rate2*rate3*rate4 | 0.419884620 | 0.14027947 | 2.99 | 0.0029 |
| rate5 | 1.704279771 | 0.40908459 | 4.17 | <.0001 |
| R-squared=.98 | | | | |

The algorithm, therefore, has the following form when the estimators are employed with two significant decimal places:

$$PC = 0.96 ISF_0 + 3.56 rate_2 + 1.03 (rate_2 * rate_1) + 2.1\ 1 rate_3 + 0.72 (rate_1 * rate_3)$$

$$+ 0.99 rate_4 + 1.14 (rate_2 * rate_4) + 0.42 (rate_2 * rate_2 * rate_4) + 1.70 rate_5.$$

One skilled in the art will recognize that the above equation is of the form of Eqn. 1 above with:

$i=0$
$k=1$
$j = 2, 3, 4$ and $5$
and
interaction terms = $rate_2 * rate_1$, $rate_1 * rate_3$, $rate_2 * rate_4$, and $rate_2 * rate_2 * rate_4$.

A Clarke Error Grid analysis can be employed to determine the accuracy and suitability of an algorithm for the prediction of a subject's blood glucose concentration. The error grid of such an analysis categorizes an analytical device's response against a reference value into one of five (5) clinical accuracy zones (i.e., zones A-E). Zone A indicates clinically accurate results, zone B indicates results that are not clinically accurate but pose minimal risk to patient health, and zones C through E indicate clinically inaccurate results that pose increasing potential risk to patient health (see Clarke, William L. et al., *Evaluating Clinical Accuracy of Systems for Self-Monitoring of Blood Glucose,* Diabetes Care, Vol. 10 No. 5, 622-628 [1987]). An effective and accurate blood glucose monitoring device should have greater than approximately 85-90% of the data in the A and B zones of the Clark Error Grid analysis, with a majority of the data in the A zone (Clark et al., supra).

A Clarke Error Grid Analysis for the prediction of a subject's blood glucose concentration based solely on a single measurement of the subject's ISF glucose concentration is depicted in FIG. 2. FIG. 3 is a Clarke Error Grid Analysis for the prediction of a subject's blood glucose concentration based on a series of ISF glucose concentrations and the algorithm immediately above. Both FIG. 3 and FIG, 4 were obtained using the data set described above.

Referring to FIGs. 2 and 3, it is evident that use of a series of ISF glucose concentrations and the algorithm above beneficially increased the percentage of predicted blood glucose concentrations in zone A to 88.2% compared to 79.5% when a sole ISF glucose concentration was employed to predict blood glucose concentration.

**Example 2: Predictive Algorithm for a Glucose Analytical Device Utilizing $ISF_i^k$, $rate_j$, $ma_nrate_m^p$, Significant Interaction Terms**

Employing the same data set as in Example 1 above, algorithms employing $ISF_i^k$, $rate_j$, $ma_nrate_m^p$, significant interaction terms were developed as described below. The algorithms employed smoothing variables of the general form $ma_nrate_m$ (discussed above) using two to four point moving averages. Weighting variables were also included to improve the algorithms' ability to accurately predict blood glucose concentration from the series of ISF glucose concentrations. The weighting algorithm used was as follows (in SAS® code):

```
weight4=ISF**-4;
newweight=200;
if ma1rate1 < 0 and ma3rate1 <= 0 then do;
      if rate1 <= 0 then newweight=weight4*(-1*rate1+1)**2;
      if rate1 > 0 then newweight=(weight4*(abs(malrate1)+1)**2)/(1+rate1);
end;
if malrate1 > 0 and ma3rate1 >= 0 then do;
      if rate1 >= 0 then newweight=weight4*(1*rate1+1)**2;
      if rate1 < 0 then newweight=(weight4*(abs(ma1rate1)+1)**2)/(1+ abs(ratel));
end;
if ma1rate1 <= 0 and ma3rate 1 > 0 then do;
      if rate1 >= 0 then newweight=(weight4*(1*rate1+1)**2)/4;
      if rate1 < 0 then newweight=(weight4*(-1*rate1+1)**2)/2;
end;
if ma1rate1 >= 0 and ma3rate1 < 0 then do;
      if rate1 > 0 then newweight=(weight4*(1*rate 1 +1)**2)/2;
      if rate1 <=0 then newweight=(weight4*(-1*rate1+1)**2)/4;
end;
if newweight ne 200 then do;
      newweight=10000000000*newweight;
end;
```

Separate equations were developed for increasing (rising) and decreasing (falling) ISF glucose concentration trends in order to provide analytical devices and methods of superior accuracy. For data series that indicate a decreasing (falling) ISF glucose concentrations, the following model was obtained by least squares regression analysis using SAS® version 8.02 and N=278 data points:

$$PC = 8.23ma1rate1 + 0.88ISF_3 + 12.04ma1rate2 + 10.54rate1 + 1.71rate1*rate2 -$$

$$0.056ISF*rate1 + 0.71(rate1)^2 + 0.68(rate2)^2 + 0.0014(ISF)^2 - 0.0011(ISF_3)^2$$

For data series that indicate an increasing (rising) ISF glucose concentration, the following model was obtained by least squares regression analysis with SAS® version 8.02 and N=180 data points:

$$PC = 4.13ISF - 1.51ISF_1 - 1.69ISF_3 - 37.06ma1rate2 + 13.67ma3rate1 - 28.35rate1 -$$

$$3.56rate1*rate2 + 0.10ISF*rate1 + 0.15ISF*rate2 + 0.47rate1*rate2*rate3 -$$

$$1.13(rate3)^2 - 0.0061(ISF)^2 + 0.0060(ISF_2)^2$$

FIG. 4 is a Clarke Error Grid Analysis for the prediction of a subject's blood glucose concentration based on a series of ISF glucose concentrations and the algorithms immediately above. FIG. 4 was obtained using the data set described above with respect to Example 1.

Another measure of device accuracy is the mean absolute % error (MPE(%)) which is determined from the mean of individual % error (PE) given by the following function:

$$PE = (PG_t\text{-}BG_t)/BG_t$$

where:

BG$_t$ = the reference glucose measurement at time t, and
PG$_t$ = the predicted glucose measurement at time t.

The MPE(%) results for the use of no algorithm (i.e., simply predicting that subject's blood glucose concentration is equal to a subject's ISF glucose concentration) and the two algorithms described immediately above are depicted in Table 1 along with selected results from FIG. 4.

Yet another measure of device accuracy is average percent bias (Avg Bias(%)). Bias (%) is determined by the following equation:

$$Bias(\%) = [(PG_t\text{-}BG_t)/BG_t]*100$$

$$Avg\ Bias(\%) = [sum\ of\ all\ Bias(\%)]/total\ number\ of\ measurements$$

Effective measurements should have an Avg Bias(%) of about 10% or less. Table 1 shows that the Avg Bias (%) criterion is beneficially decreased by use of the predictive algorithm.

The correlation between calculated and measured blood glucose values was also assessed. The correlation coefficient values (R) also presented in Table 1 below. Effective measurements should have R values of greater than about 0.85. As can be seen, the predictive algorithm of the present invention provides for improved correlation between actual and predicted values.

TABLE 1

| Algorithms | N | MPE(%) | A (%) | B (%) | Other (%) | R | Avg Bias (%) |
|---|---|---|---|---|---|---|---|
| None | 458 | 14 | 79.9 | 17.7 | 2.4 | 0.94 | 6.76 |
| Example 2 | 458 | 10 | 88.4 | 9.6 | 2.0 | 0.96 | 0.46 |

FIG. 5 is a flow chart illustrating a sequence of steps in a process 500 for predicting a subject's blood analyte concentration based on the subjects' ISF analyte concentration according to an exemplary embodiment of the present invention. Process 500 includes extracting a plurality of interstitial fluid (ISF) samples from a subject in a sequential manner, as set forth in step 510, and sequentially determining an ISF analyte concentration in each of the plurality of ISF samples, as set forth in step 520. The result of step 520 is the creation of a series of ISF analyte concentrations.

Steps 510 and 520 of process 500 can be accomplished using any suitable techniques including those described above with respect to sampling modules and analysis modules of analytical devices according to the present invention.

Next, the subject's blood analyte concentration is predicted based on the series of ISF analyte concentrations by performing at least one algorithm of the form(s) described above with respect to analytical devices according to the present invention, as set forth in step 530.

While preferred embodiments of the present invention have been shown and described herein, it will be obvious to those skilled in the art that such embodiments are provided by way of example only. Numerous variations, changes, and substitutions will now occur to hose skilled in the art without departing from the invention.

It should be understood that various alternatives to the embodiments of the invention described herein may be employed in practicing the invention. It is intended that the following claims define the scope of the invention and that methods and structures within the scope of these claims and their equivalents be covered thereby.

**Claims**

1. An analytical device for predicting a subject's whole blood analyte concentration based on the subject's interstitial fluid analyte concentration, the analytical device comprising:

an interstitial fluid sampling module for extracting a plurality of interstitial fluid (ISF) samples from a subject in a sequential manner;

an analysis module for sequentially determining an ISF analyte concentration in each of the plurality of ISF samples, thereby creating a series of ISF analyte concentrations; and

a prediction module for storing the series of ISF analyte concentrations and predicting the subject's whole blood analyte concentration based on the series of ISF analyte concentrations by performing at least one algorithm of the following general form:

$$PC = f (ISF_i^k, rate_j, \text{significant interaction terms})$$

where:

PC = the predicted subject's whole blood analyte concentration;

i is an integer with predetermined values selected from the values of 0, 1,2, 3,4 and 5;

j is an integer with predetermined values selected from the values of 1, 2, 3, 4 and 5;

k is an integer with predetermined values selected from the values of 1 and 2;

$ISF_i$ is a measured ISF analyte concentration in the series of ISF analyte concentrations;

$rate_j$ is a rate of change between immediately adjacent ISF analyte concentrations in the series of ISF analyte concentrations; and

significant interaction terms = statistically significant interaction terms involving terms selected from the group consisting of $ISF_i^k$ and $rate_j$.

2. The analytical device of claim 1, wherein i = 0, k = 1, j = 2, 3, 4 and 5 and interaction terms = $rate_2*rate_1$, $rate_1*rate_3$, $rate_2*rate_4$, and $rate_2*rate_2*rate_4$.

3. The analytical device of claim 1, wherein the analyte is glucose.

4. The analytical device of claim 1, wherein the predicting the subject's whole blood analyte concentration is based on the series of ISF analyte concentrations by performing at least one algorithm of the following general form:

$$PC = f (ISF_i^k, rate_j, ma_n rate_m^p, \text{significant interaction terms})$$

where:

p is an integer with predetermined values selected from the values of 1 and 2;

n and m are integers with predetermined values selected from the values of 1, 2 and 3;

$ma_n rate_m$ is the moving average rate between adjacent averages of groupings of ISF values; and

significant interaction terms = statistically significant interaction terms involving terms selected from the group consisting of $ISF_i^k$, $rate_j$, and $ma_n rate_m^p$.

5. The analytical device of claim 4, wherein the prediction module predicts the subject's whole blood analyte concentration by determining whether the series of ISF analyte concentrations is indicative of a rising ISF analyte concentration or a falling ISF analyte concentration, selecting an algorithm based on the determination and performing the selected algorithm.

6. The analytical device of claim 5, wherein the prediction module predicts the subject's whole blood analyte concentration by determining whether the series of ISF analyte concentrations is indicative of a rising ISF analyte concentration or a falling ISF analyte concentration based on an $ma_n rate_m$.

7. The analytical device of claim 6, wherein the algorithm employed for a falling ISF analyte concentration is:

$$PC = 8.23ma1rate1 + 0.88ISF_3 + 12.04ma1rate2 + 10.54rate1 +$$

$$1.71rate1*rate2 - 0.056ISF*rate1 + 0.71(rate1)^2 + 0.68(rate2)^2 +$$

$$0.0014(ISF)^2 \_sq-0.0011 (ISF_3)^2.$$

8.  The analytical device of claim 6, wherein the algorithm employed for a rising ISF analyte concentration is:

$$PC = 4.13ISF - 1.51ISF_1 - 1.69ISF_3 - 37.06malrate2 + 13.67ma3rate1 -$$

$$28.35rate1 - 3.56rate1*rate2 + 0.10ISF*rate1 + 0.15ISF*rate2 + 0.47rate1*rate2*rate3 -$$

$$1.13(rate3)^2 - 0.0061(ISF)^2 + 0.0060(ISF_2)^2.$$

9.  The analytical device of claim 4, wherein the analyte is glucose.

10. The analytical device of claim 1, wherein the series of ISF analyte concentrations includes five ISF analyte concentrations.

11. The analytical device of claim 1, wherein the sampling module extracts the plurality of ISF samples at a time interval in the range of five to fifteen minutes.

12. A method for predicting a subject's whole blood analyte concentration based on the subject's interstitial fluid analyte concentration, the method comprising:

> extracting a plurality of interstitial fluid (ISF) samples from a subject in a sequential manner;
> sequentially determining an ISF analyte concentration in each of the plurality of ISF samples, thereby creating a series of ISF analyte concentrations; and
> predicting the subject's blood analyte concentration based on the series of ISF analyte concentrations by performing at least one algorithm of the following form:

$$PC = f (ISF_i^k, rate_j, \text{significant interaction terms})$$

> where:

> PC = the predicted subject's whole blood analyte concentration;
> i is an integer with predetermined values selected from the values of 0, 1, 2, 3, 4 and 5;
> j is an integer with predetermined values selected from the values of 1, 2, 3, 4 and 5;
> k is an integer with predetermined values selected from the values of 1 and 2;
> $ISF_i$ is a measured ISF analyte concentration in the series of ISF analyte concentrations;
> $rate_j$ is the rate of change between adjacent ISF analyte concentrations in the series of ISF analyte concentrations; and
> significant interaction terms = statistically significant interaction terms involving terms selected from the group consisting of $ISF_i^k$ and $rate_j$.

13. The method of claim 12, wherein the predicting set employs an algorithm of the form:

$$PC = f (ISF_i^k, rate_j, ma_n rate_m^p, \text{significant interaction terms})$$

> where:

> p is an integer with predetermined values selected from the values of 1 and 2;
> m and n are integers with predetermined values selected from the values of 1, 2 and 3;
> $ma_n rate_m$ is the moving average rate between adjacent averages of groupings of ISF values; and
> significant interaction terms = statistically significant interaction terms involving terms selected from the group consisting of $ISF_i^k$, $rate_j$, and $ma_n rate_m^p$.

14. The method of claim 13, wherein the predicting step predicts the subject's whole blood analyte concentration by determining whether the series of ISF analyte concentrations is indicative of a rising ISF analyte concentration or a falling ISF analyte concentration, selecting the algorithm based on the determination and performing the selected algorithm.

**15.** The method of claim 13, wherein the predicting step predicts the subject's whole blood analyte concentration by determining whether the series of ISF analyte concentrations is indicative of a rising ISF analyte concentration or a falling ISF analyte concentration based on an $ma_n rate_m$.

**16.** The method of claim 12, wherein the extracting step extracts a plurality of ISF samples from a subject's dermis.

EP 1 510 171 A1

European Patent
Office

EUROPEAN SEARCH REPORT

Application Number

EP 04 25 5207

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | EP 1 260 815 A (LIFESCAN INC) 27 November 2002 (2002-11-27) * column 9, lines 46-55 * * column 7, lines 8-15 * * column 3, paragraph 13 - column 5, paragraph 19 * | 1,3, 10-12,16 | A61B5/00 A61B5/15 |
| A | | 2,4-9, 13-15 | |
| Y | SCHMIDTKE ET AL.: "Measurement and modeling of the transient difference between blood and subcutaneous glucose concentrations in the rat after injection of insulin" PROCEEDINGS OF THE NATIONAL ACADEMY OF SCIENCES OF THE UNITED STATES OF AMERICA, vol. 95, January 1998 (1998-01), pages 294-299, XP001203807 * page 296, column 1 - page 298, column 1 * | 1,3, 10-12,16 | |
| A | US 2003/055326 A1 (SOHRAB BORZU) 20 March 2003 (2003-03-20) * paragraphs [0015] - [0027] * | 1,12 | TECHNICAL FIELDS SEARCHED (Int.Cl.7) A61B |
| A | US 6 332 871 B1 (RADWANSKI RYSZARD ET AL) 25 December 2001 (2001-12-25) * column 3, line 30 - column 8, line 40 * | 1,12 | |

The present search report has been drawn up for all claims

| Place of search | Date of completion of the search | Examiner |
|---|---|---|
| Munich | 10 November 2004 | Rivera Pons, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**EP 1 510 171 A1**

### ANNEX TO THE EUROPEAN SEARCH REPORT
### ON EUROPEAN PATENT APPLICATION NO.

EP 04 25 5207

This annex lists the patent family members relating to the patent documents cited in the above-mentioned European search report.
The members are as contained in the European Patent Office EDP file on
The European Patent Office is in no way liable for these particulars which are merely given for the purpose of information.

10-11-2004

| Patent document cited in search report | | Publication date | Patent family member(s) | | Publication date |
|---|---|---|---|---|---|
| EP 1260815 | A | 27-11-2002 | US | 2002177764 A1 | 28-11-2002 |
| | | | AU | 4439402 A | 28-11-2002 |
| | | | CA | 2387287 A1 | 25-11-2002 |
| | | | CN | 1388376 A | 01-01-2003 |
| | | | CZ | 20021820 A3 | 16-04-2003 |
| | | | EP | 1260815 A2 | 27-11-2002 |
| | | | JP | 2003024284 A | 28-01-2003 |
| | | | PL | 354134 A1 | 02-12-2002 |
| | | | US | 2003158472 A1 | 21-08-2003 |
| | | | US | 2004162473 A1 | 19-08-2004 |
| US 2003055326 | A1 | 20-03-2003 | US | 2002188185 A1 | 12-12-2002 |
| | | | AU | 4445102 A | 19-12-2002 |
| | | | CA | 2390332 A1 | 12-12-2002 |
| | | | CN | 1391104 A | 15-01-2003 |
| | | | CZ | 20022024 A3 | 17-12-2003 |
| | | | EP | 1266625 A2 | 18-12-2002 |
| | | | JP | 2003038464 A | 12-02-2003 |
| | | | PL | 354419 A1 | 16-12-2002 |
| US 6332871 | B1 | 25-12-2001 | US | 2002082522 A1 | 27-06-2002 |
| | | | AU | 3070397 A | 05-12-1997 |
| | | | AU | 3131097 A | 05-12-1997 |
| | | | AU | 3206797 A | 09-12-1997 |
| | | | AU | 3207197 A | 05-12-1997 |
| | | | AU | 3284797 A | 05-12-1997 |
| | | | AU | 3368297 A | 05-12-1997 |
| | | | DE | 19781044 T0 | 03-12-1998 |
| | | | DE | 19781046 T0 | 24-12-1998 |
| | | | DE | 19781097 T0 | 01-07-1999 |
| | | | DE | 19781098 T0 | 24-12-1998 |
| | | | DE | 29723357 U1 | 15-10-1998 |
| | | | DE | 29723371 U1 | 06-08-1998 |
| | | | DK | 64698 A | 18-01-1999 |
| | | | DK | 64798 A | 18-01-1999 |
| | | | DK | 67498 A | 18-01-1999 |
| | | | EP | 0904022 A1 | 31-03-1999 |
| | | | EP | 0906062 A1 | 07-04-1999 |
| | | | EP | 0955914 A1 | 17-11-1999 |
| | | | EP | 0955909 A1 | 17-11-1999 |
| | | | ES | 2121564 A1 | 01-05-2000 |
| | | | ES | 2121565 A1 | 01-05-2000 |
| | | | GB | 2322561 A ,B | 02-09-1998 |
| | | | GB | 2323792 A ,B | 07-10-1998 |
| | | | GB | 2322562 A ,B | 02-09-1998 |
| | | | GB | 2325167 A ,B | 18-11-1998 |

**ANNEX TO THE EUROPEAN SEARCH REPORT
ON EUROPEAN PATENT APPLICATION NO.**

EP 04 25 5207

This annex lists the patent family members relating to the patent documents cited in the above-mentioned European search report.
The members are as contained in the European Patent Office EDP file on
The European Patent Office is in no way liable for these particulars which are merely given for the purpose of information.

10-11-2004

| Patent document cited in search report | Publication date | Patent family member(s) | Publication date |
|---|---|---|---|
| US 6332871 B1 | | IL 124426 A | 01-06-2000 |
| | | JP 3356782 B2 | 16-12-2002 |
| | | JP 2002503118 T | 29-01-2002 |
| | | JP 2002502271 T | 22-01-2002 |
| | | JP 2000511068 T | 29-08-2000 |
| | | JP 3494660 B2 | 09-02-2004 |
| | | JP 2002503119 T | 29-01-2002 |
| | | JP 2003102711 A | 08-04-2003 |
| | | JP 2003102712 A | 08-04-2003 |
| | | WO 9743962 A1 | 27-11-1997 |
| | | WO 9742882 A1 | 20-11-1997 |
| | | WO 9742883 A1 | 20-11-1997 |
| | | WO 9742885 A1 | 20-11-1997 |
| | | WO 9742886 A1 | 20-11-1997 |
| | | WO 9742888 A1 | 20-11-1997 |
| | | US 6015392 A | 18-01-2000 |
| | | US 6056701 A | 02-05-2000 |
| | | US 6183489 B1 | 06-02-2001 |
| | | US 6099484 A | 08-08-2000 |
| | | US 6352514 B1 | 05-03-2002 |
| | | US 6319210 B1 | 20-11-2001 |
| | | US 2004073140 A1 | 15-04-2004 |
| | | US 2004006285 A1 | 08-01-2004 |

EPO FORM P0459

For more details about this annex : see Official Journal of the European Patent Office, No. 12/82